# EUROPEAN PATENT SPECIFICATION

(11) **EP 4 186 427 B1**
(45) Date of publication and mention of the grant of the patent: **03.09.2025**
(21) Application number: 22201610.7
(22) Date of filing: 14.10.2022
(51) Int. Cl.: A61B 6/00, A61B 8/08, A61B 8/00

(54) **ULTRASOUND IMAGE ANALYSIS APPARATUS, ULTRASOUND DIAGNOSTIC APPARATUS, AND CONTROL METHOD FOR ULTRASOUND IMAGE ANALYSIS APPARATUS**
ULTRASCHALLBILDANALYSEVORRICHTUNG, ULTRASCHALLDIAGNOSEVORRICHTUNG UND STEUERUNGSVERFAHREN FÜR ULTRASCHALLBILDANALYSEVORRICHTUNG
APPAREIL D'ANALYSE D'IMAGE ULTRASONORE, APPAREIL DE DIAGNOSTIC ULTRASONORE ET PROCÉDÉ DE COMMANDE POUR APPAREIL D'ANALYSE D'IMAGE ULTRASONORE

(30) Priority: 25.11.2021 JP 2021191272
(43) Date of publication of application: 31.05.2023
(73) Proprietor: FUJIFILM Corporation, Tokyo 106-8620 (JP)
(72) Inventor: KOSHINO, Riko, Tokyo, 106-8620 (JP)
(74) Representative: Dehns Germany Partnerschaft mbB

(56) References cited:
- US-A1- 2010 014 738
- US-A1- 2020 375 562
- CRYSTAL: "Using Sonography to Screen Women with Mammographically Dense Breasts", 1 January 2003 (2003-01-01), XP093036621, Retrieved from the Internet <URL:https://www.ajronline.org/doi/epdfplus/10.2214/ajr.181.1.1810177> [retrieved on 20230331]
- IKEDO YUJI ET AL: "Automated analysis of breast parenchymal patterns in whole breast ultrasound images: preliminary experience", INTERNATIONAL JOURNAL OF COMPUTER ASSISTED RADIOLOGY AND SURGERY, vol. 4, no. 3, 14 March 2009 (2009-03-14), DE, pages 299 - 306, XP093036689, ISSN: 1861-6410, Retrieved from the Internet <URL:http://link.springer.com/article/10.1007/s11548-009-0295-0/fulltext.html> DOI: 10.1007/s11548-009-0295-0
- LEE SU HYUN ET AL: "Glandular Tissue Component and Breast Cancer Risk in Mammographically Dense Breasts at Screening Breast US", RADIOLOGY, vol. 301, no. 1, 1 October 2021 (2021-10-01), US, pages 57 - 65, XP093036140, ISSN: 0033-8419, DOI: 10.1148/radiol.2021210367

## Description

### BACKGROUND OF THE INVENTION

### 1. Field of the Invention

The present invention relates to an ultrasound image analysis apparatus that performs analysis on an ultrasound image obtained by imaging a breast of a subject.

In addition, the present invention also relates to an ultrasound diagnostic apparatus including the ultrasound image analysis apparatus and a control method for the ultrasound image analysis apparatus.

### 2. Description of the Related Art

In related art, an ultrasound diagnostic apparatus using ultrasound images has been put into practical use in a medical field. In general, an ultrasound diagnostic apparatus includes an ultrasound probe in which a transducer array is provided and an apparatus main body connected to the ultrasound probe. An ultrasound beam is transmitted from the ultrasound probe toward a subject, an ultrasound echo from the subject is received by the ultrasound probe, and a reception signal is electrically processed. Thereby, an ultrasound image is generated.

A composition of a fat tissue and a mammary gland tissue in a breast varies from person to person, while an anatomical structure of a breast is common. In the mammary gland tissue, a main mammary duct branches into extralobular mammary ducts, which are connected to numerous lobules. A stroma is present around the lobules, and the mammary gland tissue includes the stroma.

It is known that there are two types of stromata around lobules: a peripheral stroma and an edematous stroma. The peripheral stroma is present along a structure from the lobule to the mammary duct, and includes many collagen fibers. On the other hand, the edematous stroma fills a space between the peripheral stromata. In the edematous stroma, substrates are rich, and collagen fibers, fat, and the like coexist. The edematous stroma includes less collagen fibers as compared with the peripheral stroma.

In recent years, a concept of risk management for individual patients has spread. On the other hand, it is known that a ratio of a mammary gland region in a breast, particularly, a high density of mammary glands, is a cancer risk factor. The ratio of the mammary gland region in a breast can be measured by using a mammography apparatus.

In addition, in Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case where a ratio of glandular tissue component (GTC) region including mammary ducts, lobules, and peripheral stromata in the mammary gland region is high even though the mammary gland region is almost the same. That is, in addition to the ratio of the mammary gland region in the breast, a ratio of the GTC region in the mammary gland region can be a risk factor. This implies a higher risk in patients with less advanced lobular retraction.

However, in the mammography apparatus, the peripheral stroma and the edematous stroma cannot be distinguished, and the entire mammary gland tissue is observed as whitish. As a result, a ratio of the GTC region in the mammary gland region cannot be measured.

JP2020-18694A discloses an ultrasound diagnostic apparatus that extracts a mammary gland region by detecting a boundary in a depth direction of an ultrasound image and detects a lesion portion existing in the mammary gland region.

US 2020/375562 discloses a radiography system, a medical imaging system, a control method, and a control program.

Crystal et al.: "Using Sonography to Screen Women with Mammographically Dense Breast", 1 January 2003 (2003-01-01) discloses sonography as a second-line screening test.

US 2010/014738 discloses a method and system for breast cancer screening.

### SUMMARY OF THE DISCLOSURE

However, the ultrasound diagnostic apparatus disclosed in JP2020-18694A aims to detect a lesion portion in the mammary gland region, and is not interested in dividing the mammary gland region into finer tissues. As a result, there is a problem that a cancer risk of the mammary gland region cannot be estimated.

In addition, in a case where the mammary glands retract and the volume of the breast decreases, a fat region occurs between lobes in the mammary gland. As a result, a ratio of a mammary gland region to a breast region may decrease. In such a case, a cancer risk is low, and thus a necessity to measure a ratio of a GTC region in the mammary gland region is reduced.

The present disclosure has been made in order to solve such problems in the related art, and an object of the present invention is to provide an ultrasound image analysis apparatus capable of estimating a cancer risk of a mammary gland region based on a result of a mammography examination, as necessary, based on an ultrasound image.

Another object of the present disclosure is to provide an ultrasound diagnostic apparatus including such an ultrasound image analysis apparatus and a control method for an ultrasound image analysis apparatus.

According to an aspect of the present invention, there is provided an ultrasound image analysis apparatus as claimed in claim 1.

The breast composition information acquisition unit can acquire, as the information related to the breast composition, a mammary gland category corresponding to the subject among mammary gland categories classified into a plurality of types according to a mammary gland density. In this case, preferably, the analysis determination unit determines to perform analysis on the glandular tissue component region in a case where the mammary gland category corresponding to the subject belongs to at least one mammary gland category which is determined in order in which the mammary gland density is high among the mammary gland categories classified into the plurality of types.

The breast composition information acquisition unit may acquire, as the information related to the breast composition, a ratio of the mammary gland region to a breast region of the subject. In addition, the breast composition information acquisition unit can include a breast composition information calculation unit that calculates the ratio of the mammary gland region to the breast region of the subject from a mammography image. Preferably, the analysis determination unit determines to perform analysis on the glandular tissue component region in a case where the ratio of the mammary gland region to the breast region is higher than a predetermined mammary gland region threshold value.

Further, the breast composition information acquisition unit may acquire, as the information related to the breast composition, a ratio of a mammary gland mass to the mammary gland region of the subject. In this case, preferably, the analysis determination unit determines to perform analysis on the glandular tissue component region in a case where the ratio of the mammary gland mass to the mammary gland region is higher than a predetermined mammary gland mass threshold value.

The mammary gland region detection unit can detect the mammary gland region by image recognition of the ultrasound image. In this case, the ultrasound image analysis apparatus may further include a breast region detection unit that detects a breast region located between a skin and a pectoralis major muscle from the ultrasound image. The mammary gland region detection unit may recognize a front boundary line and a rear boundary line in the breast region detected by the breast region detection unit, and detect a region between the front boundary line and the rear boundary line, as the mammary gland region.

Alternatively, the mammary gland region detection unit can detect the mammary gland region from the ultrasound image by using deep learning.

The glandular tissue component region extraction unit can extract the glandular tissue component region by binarizing the mammary gland region of the ultrasound image by using a brightness threshold value.

Further, the glandular tissue component region extraction unit can extract the glandular tissue component region from the mammary gland region of the ultrasound image by using deep learning.

The glandular tissue component region ratio calculation unit can calculate the ratio of the glandular tissue component region to the mammary gland region based on the number of occupied pixels of the mammary gland region and the number of occupied pixels of the glandular tissue component region in the ultrasound image.

The ultrasound image may be a three-dimensional ultrasound image. The glandular tissue component region ratio calculation unit may calculate the ratio of the glandular tissue component region to the mammary gland region based on a volume of the mammary gland region detected by the mammary gland region detection unit and a volume of the glandular tissue component region extracted by the glandular tissue component region extraction unit.

According to another aspect of the present invention, there is provided an ultrasound diagnostic apparatus including: a monitor that displays an ultrasound image; and the ultrasound image analysis apparatus, in which the glandular tissue component region analysis unit displays an analysis result of the glandular tissue component region on the monitor.

Preferably, the glandular tissue component region analysis unit stores the analysis result of the glandular tissue component region in a tag associated with the ultrasound image.

Preferably, the ultrasound diagnostic apparatus further includes: an ultrasound probe; and an image generation unit that generates the ultrasound image obtained by imaging the breast of the subject by transmitting and receiving an ultrasound beam to and from the subject using the ultrasound probe.

According to another aspect of the present invention, there is provided a control method as claimed in claim 19.

According to the present disclosure, the breast composition information acquisition unit acquires information related to a breast composition of a subject obtained by a mammography examination. In addition, the analysis determination unit determines whether or not to perform analysis on a glandular tissue component region including mammary ducts, lobules, and peripheral stromata in a mammary gland region based on the information related to the breast composition of the subject. In addition, the glandular tissue component region analysis unit performs analysis on the glandular tissue component region based on an ultrasound image obtained by imaging a breast of the subject in a case where it is determined to perform analysis. Therefore, it is possible to estimate a cancer risk of a mammary gland region based on a result of a mammography examination, as necessary, based on an ultrasound image.

### BRIEF DESCRIPTION OF THE DRAWINGS

Fig. 1 is a block diagram illustrating a configuration of an ultrasound diagnostic apparatus according to an embodiment of the present invention.
Fig. 2 is a block diagram illustrating an internal configuration of a transmission/reception circuit according to the embodiment.
Fig. 3 is a block diagram illustrating an internal configuration of an image generation unit according to the embodiment.
Fig. 4 is a block diagram illustrating an internal configuration of a breast composition information calculation unit according to the embodiment.
Fig. 5 is a block diagram illustrating an internal configuration of a GTC region analysis unit according to the embodiment.
Fig. 6 is a diagram illustrating an ultrasound image obtained by imaging a mammary gland region of a subject.
Fig. 7 is a diagram illustrating an ultrasound image including a mammary gland region in which a GTC region is imaged.
Fig. 8 is a diagram illustrating a binarized image obtained by binarizing the mammary gland region by using a brightness threshold value.
Fig. 9 is a block diagram illustrating a configuration of a mammography apparatus connected to the ultrasound diagnostic apparatus via a network.
Fig. 10 is a flowchart illustrating an operation according to the embodiment.
Fig. 11 is a flowchart illustrating an operation in analysis determination processing.
Fig. 12 is a flowchart illustrating an operation in GTC region analysis processing.

### DESCRIPTION OF THE PREFERRED EMBODIMENTS

Hereinafter, embodiments of the present invention will be described with reference to the accompanying drawings.

A description of components to be described below is based on a representative embodiment of the present invention. On the other hand, the present invention is not limited to such an embodiment.

Note that, in this specification, a numerical range represented by using "to" means a range including numerical values described before and after "to", both ends inclusive, as a lower limit value and an upper limit value.

In this specification, it is assumed that terms "identical" and "same" include an error margin which is generally allowed in the technical field.

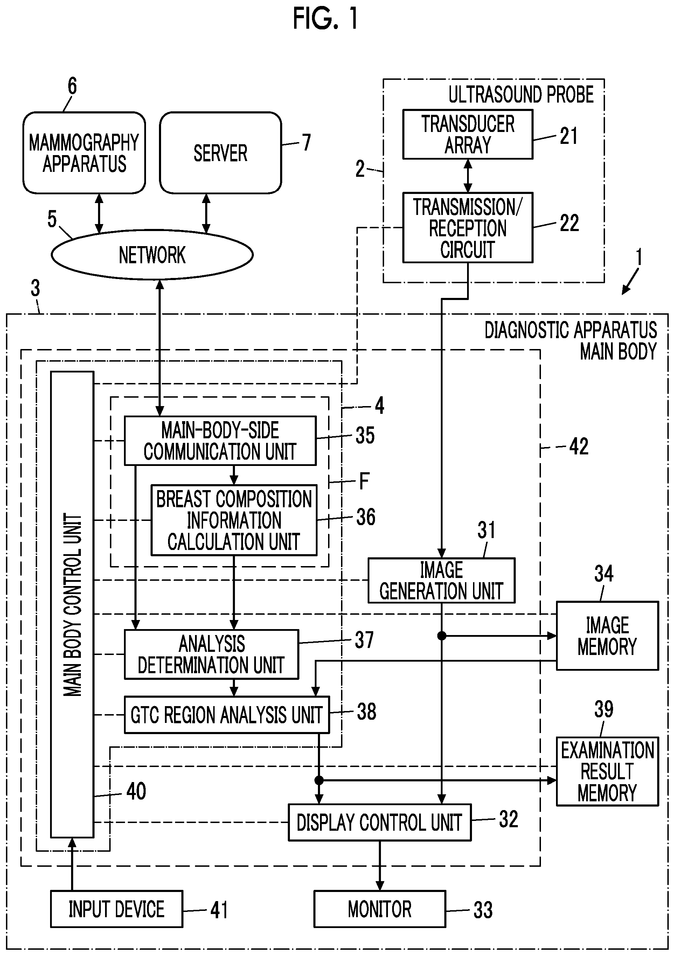

Fig. 1 illustrates a configuration of an ultrasound diagnostic apparatus 1 according to an embodiment of the present invention. The ultrasound diagnostic apparatus 1 includes an ultrasound probe 2 and a diagnostic apparatus main body 3. The ultrasound probe 2 and the diagnostic apparatus main body 3 are wired-connected to each other via a cable (not illustrated).

The ultrasound probe 2 includes a transducer array 21 and a transmission/reception circuit 22 connected to the transducer array 21.

The diagnostic apparatus main body 3 includes an image generation unit 31 connected to the transmission/reception circuit 22 of the ultrasound probe 2. A display control unit 32 and a monitor 33 are sequentially connected to the image generation unit 31, and an image memory 34 is connected to the image generation unit 31.

In addition, the diagnostic apparatus main body 3 includes a main-body-side communication unit 35. A breast composition information calculation unit 36 is connected to the main-body-side communication unit 35, and an analysis determination unit 37 is connected to the main-body-side communication unit 35 and the breast composition information calculation unit 36.

Further, a glandular tissue component (GTC) region analysis unit 38 is connected to the image memory 34 and the analysis determination unit 37, and an examination result memory 39 and a display control unit 32 are connected to the GTC region analysis unit 38.

A main body control unit 40 is connected to the image generation unit 31, the display control unit 32, the image memory 34, the main-body-side communication unit 35, the breast composition information calculation unit 36, the analysis determination unit 37, the GTC region analysis unit 38, and the examination result memory 39. An input device 41 is connected to the main body control unit 40. In addition, the transmission/reception circuit 22 of the ultrasound probe 2 is connected to the main body control unit 40.

A processor 42 is configured by the image generation unit 31, the display control unit 32, the main-body-side communication unit 35, the breast composition information calculation unit 36, the analysis determination unit 37, the GTC region analysis unit 38, and the main body control unit 40.

In addition, an ultrasound image analysis apparatus 4 is configured by the main-body-side communication unit 35, the breast composition information calculation unit 36, the analysis determination unit 37, the GTC region analysis unit 38, and the main body control unit 40 in the processor 42.

Further, a mammography apparatus 6 and a server 7 are connected to the main-body-side communication unit 35 via a network 5.

The transducer array 21 of the ultrasound probe 2 includes a plurality of ultrasound transducers which are one-dimensionally or two-dimensionally arranged. Each of these transducers transmits an ultrasound wave according to a drive signal supplied from the transmission/reception circuit 22, receives a reflected wave from a subject, and outputs an analog reception signal. Each transducer is configured by, for example, forming electrodes on both ends of a piezoelectric body such as a piezoelectric ceramic represented by lead zirconate titanate (PZT), a polymeric piezoelectric element represented by poly vinylidene di fluoride (PVDF), or a piezoelectric single crystal represented by a lead magnesium niobate-lead titanate solid solution (PMN-PT).

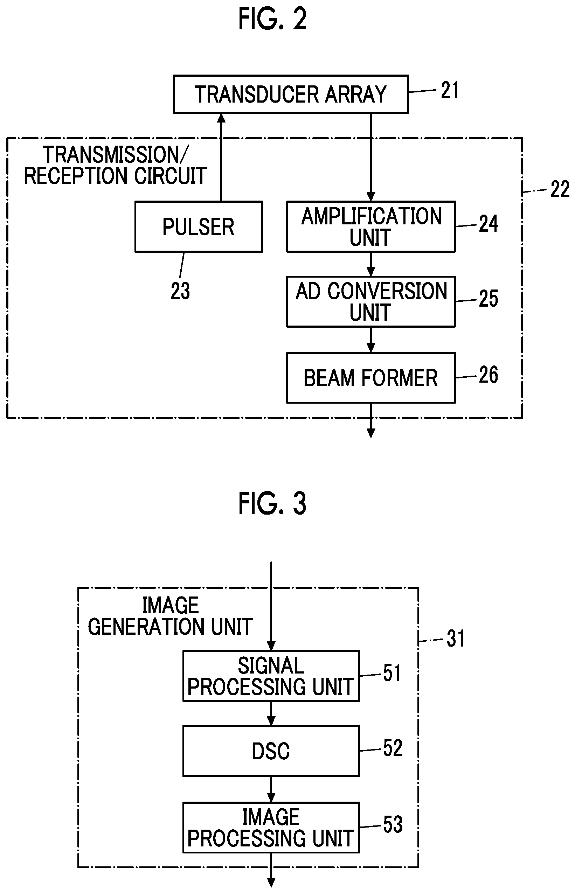

The transmission/reception circuit 22 transmits an ultrasound wave from the transducer array 21 and generates a sound ray signal based on the reception signal acquired by the transducer array 21 under a control of the main body control unit 40. As illustrated in Fig. 2, the transmission/reception circuit 22 includes a pulser 23 connected to the transducer array 21, an amplification unit 24 sequentially connected in series to the transducer array 21, an analog digital (AD) conversion unit 25, and a beam former 26.

The pulser 23 includes, for example, a plurality of pulse generators, adjusts a delay amount of each drive signal based on a transmission delay pattern which is selected according to a control signal from the main body control unit 40 such that ultrasound waves to be transmitted from the plurality of transducers of the transducer array 21 form ultrasound beams, and supplies each drive signal with the adjusted delay amount to the plurality of transducers. In this way, in a case where a voltage having a pulse shape or a continuous wave shape is applied to the electrodes of the transducers of the transducer array 21, the piezoelectric body expands and contracts. Thereby, ultrasound waves having a pulse shape or a continuous wave shape are generated from each transducer, and thus an ultrasound beam is formed from a composite wave of these ultrasound waves.

The transmitted ultrasound beam is reflected by an object such as a portion of a subject, and an ultrasound echo propagates toward the transducer array 21 of the ultrasound probe 2. The ultrasound echo which propagates toward the transducer array 21 in this way is received by each transducer included in the transducer array 21. At this time, in a case where the propagating ultrasound echo is received, each transducer included in the transducer array 21 expands and contracts. Thereby, a reception signal as an electrical signal is generated, and these reception signals are output to the amplification unit 24.

The amplification unit 24 amplifies the signal which is input from each transducer included in the transducer array 21, and transmits the amplified signal to the AD conversion unit 25. The AD conversion unit 25 converts the signal transmitted from the amplification unit 24 into pieces of digital reception data, and transmits the pieces of reception data to the beam former 26. The beam former 26 performs so-called reception focus processing by applying and adding a delay to each of the pieces of reception data which is converted by the AD conversion unit 25 according to a sound velocity or a sound velocity distribution which is set based on a reception delay pattern selected according to a control signal from the main body control unit 40. By this reception focus processing, a sound ray signal obtained by performing phasing addition on each of the pieces of reception data which is converted by the AD conversion unit 25 and narrowing down a focus of the ultrasound echo is acquired.

As illustrated in Fig. 3, the image generation unit 31 of the diagnostic apparatus main body 3 has a configuration in which a signal processing unit 51, a digital scan converter (DSC) 52, and an image processing unit 53 are sequentially connected in series.

The signal processing unit 51 performs, on the sound ray signal transmitted from the transmission/reception circuit 22 of the ultrasound probe 2, correction of attenuation due to a distance according to a depth of a reflection position of the ultrasound wave and then performs envelope detection processing. Thereby, an ultrasound image signal (B-mode image signal), which is tomographic image information related to tissues in the subject, is generated.

The DSC 52 converts (raster-converts) the ultrasound image signal generated by the signal processing unit 51 into an image signal conforming to a normal television signal scanning method.

The image processing unit 53 performs various required image processing such as gradation processing on the ultrasound image signal which is input from the DSC 52, and then outputs a signal representing the ultrasound image to the display control unit 32 and the image memory 34. The signal representing the ultrasound image generated by the image generation unit 31 in this way is simply referred to as an ultrasound image.

Under a control of the main body control unit 40, the display control unit 32 performs predetermined processing on the ultrasound image transmitted from the image generation unit 31, and displays the ultrasound image on the monitor 33.

The monitor 33 displays the ultrasound image under a control of the display control unit 32, and includes a display device such as a liquid crystal display (LCD) or an organic electroluminescence (EL) display.

The image memory 34 is a memory that stores the ultrasound image generated by the image generation unit 31 under a control of the main body control unit 40. For example, the image memory 34 can store a plurality of frames of ultrasound images generated by the image generation unit 31 in correspondence with diagnosis on a breast of a subject.

As the image memory 34, a flash memory, a hard disc drive (HDD), a solid state drive (SSD), a flexible disc (FD), a magneto-optical (MO) disc, a magnetic tape (MT), a random access memory (RAM), a compact disc (CD), a digital versatile disc (DVD), a secure digital (SD) card, and a recording medium such as a Universal Serial Bus (USB) memory can be used.

The main-body-side communication unit 35 receives breast composition information or a mammography image that is related to a breast composition of the subject and is transmitted from the mammography apparatus 6 or the server 7 via the network 5, under a control of the main body control unit 40.

As the breast composition information, information such as a mammary gland category Cm corresponding to the subject, a mammary gland region ratio Rm1 which is a ratio of a mammary gland region to a breast region of the subject, and a mammary gland mass ratio Rm2 which is a ratio of a mammary gland mass to a mammary gland region of the subject can be used, the information being obtained by a mammography examination.

According to "Breast Imaging Reporting and Data System 5th-edition (BI-RADS 5th-edition)" which is a comprehensive guideline for breast cancer image diagnosis by American College of Radiology (ACR), it is recommended to classify the breast composition into four types of mammary gland categories C1 to C4, from a low mammary gland density to a high mammary gland density: "fatty", "scattered mammary gland", "heterogeneous high density", and "extremely high density". For example, the mammary gland category C4 of "extremely high density" and the mammary gland category C3 of "heterogeneous high density", which indicate a high mammary gland density, are considered as breast cancer risk factors. In mammography examinations, a mammary gland category Cm corresponding to a subject is often indicated as an examination result.

Therefore, the main-body-side communication unit 35 receives, as the breast composition information, the mammary gland category Cm of the subject obtained from the mammography examination in the mammography apparatus 6 from the mammography apparatus 6 or the server 7.

The mammary gland category Cm of the subject can be automatically assigned by the mammography apparatus 6, the server 7, or another workstation, or can be manually assigned by the user. The main-body-side communication unit 35 can receive the mammary gland category Cm of the subject from the mammography apparatus 6, the server 7, or another workstation.

In addition, software for analyzing a mammary gland density from a mammography image has been developed. By using the software, the mammography apparatus 6 or the server 7 can calculate a mammary gland region ratio Rm1 which is a ratio of a mammary gland region to a breast region of a subject from a mammography image. The main-body-side communication unit 35 can also receive, as the breast composition information, the mammary gland region ratio Rm1 from the mammography apparatus 6 or the server 7.

Further, for example, JP2019-177050A discloses a method of measuring a mammary gland mass ratio Rm2 which is a ratio of a mammary gland mass to a mammary gland region of a subject from a mammography image, that is, a mammary gland content of a mammary gland tissue in a mammary gland concentration region in which mammary glands are concentrated by using a volume ratio. In the method, the main-body-side communication unit 35 may receive, as breast composition information, the mammary gland mass ratio Rm2 from the mammography apparatus 6 or the server 7.

In this way, in a case where the mammary gland category Cm, the mammary gland region ratio Rm1, or the mammary gland mass ratio Rm2 is received as breast composition information from the mammography apparatus 6 or the server 7 by the main-body-side communication unit 35, the main-body-side communication unit 35 transmits the received breast composition information to the analysis determination unit 37.

In addition, the main-body-side communication unit 35 can also receive a mammography image instead of the breast composition information from the mammography apparatus 6 or the server 7. In this case, the main-body-side communication unit 35 transmits the received mammography image to the breast composition information calculation unit 36.

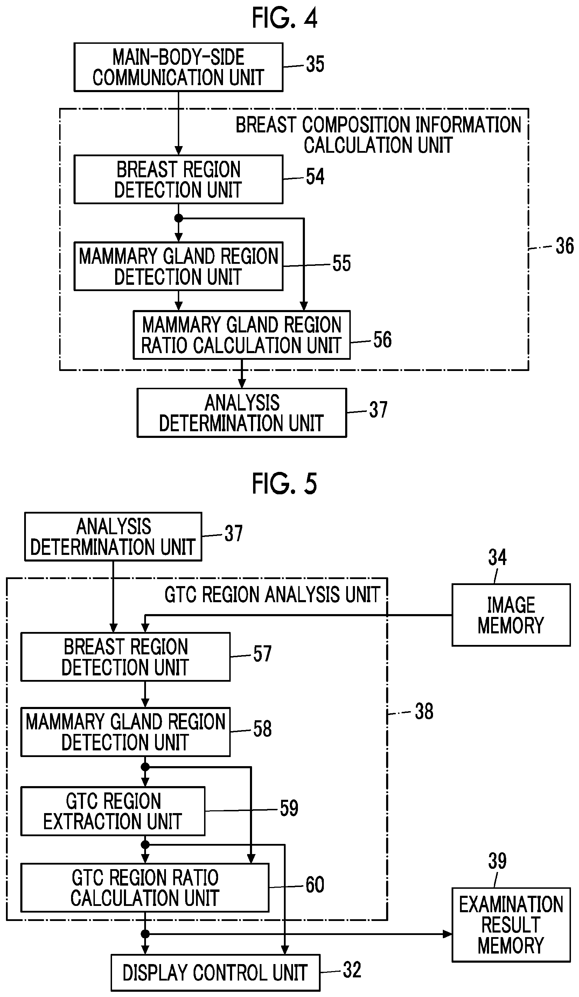

The breast composition information calculation unit 36 calculates, from the mammography image, a mammary gland region ratio Rm1 which is a ratio of a mammary gland region to a breast region of a subject. As illustrated in Fig. 4, the breast composition information calculation unit 36 includes a breast region detection unit 54 connected to the main-body-side communication unit 35, a mammary gland region detection unit 55 connected to the breast region detection unit 54, and a mammary gland region ratio calculation unit 56 connected to the breast region detection unit 54 and the mammary gland region detection unit 55.

The breast region detection unit 54 detects a breast region of the subject that is imaged in the mammography image by analyzing the mammography image received by the main-body-side communication unit 35, and the mammary gland region detection unit 55 detects a mammary gland region of the subject from the breast region of the mammography image that is detected by the breast region detection unit 54.

The mammary gland region ratio calculation unit 56 calculates a mammary gland region ratio Rm1 in the mammography image based on the breast region detected by the breast region detection unit 54 and the mammary gland region detected by the mammary gland region detection unit 55, and transmits the calculated mammary gland region ratio Rm1 as breast composition information to the analysis determination unit 37.

The breast composition information calculation unit 36 can detect a breast region and a mammary gland region from the mammography image by using a determination model learned using a machine learning technique such as deep learning, and can calculate a mammary gland region ratio Rm1 which is a ratio of the mammary gland region to the breast region of the subject.

In a case where the main-body-side communication unit 35 receives the mammography image including three-dimensional images, the mammary gland region ratio calculation unit 56 can calculate a mammary gland region ratio Rm1 from a ratio between a volume of the breast region detected by the breast region detection unit 54 and a volume of the mammary gland region detected by the mammary gland region detection unit 55.

Further, in a case where the main-body-side communication unit 35 receives the mammography image including two-dimensional images, the mammary gland region ratio calculation unit 56 may calculate a mammary gland region ratio Rm1 from a ratio between an area of the breast region detected by the breast region detection unit 54 and an area of the mammary gland region detected by the mammary gland region detection unit 55.

The main-body-side communication unit 35 and the breast composition information calculation unit 36 are included in a breast composition information acquisition unit F that acquires the breast composition information obtained in a mammography examination. The breast composition information may include the mammary gland category Cm, the mammary gland region ratio Rm1, and the mammary gland mass ratio Rm2 that are received by the main-body-side communication unit 35. The breast composition information may include the mammary gland region ratio Rm1 calculated based on the mammography image by the breast composition information calculation unit 36. The breast composition information is transmitted from the breast composition information acquisition unit F to the analysis determination unit 37.

In addition, the breast composition information calculation unit 36 can calculate not only the mammary gland region ratio Rm1 but also at least one of the mammary gland category Cm or the mammary gland mass ratio Rm2. In this case, the breast composition information including the mammary gland category Cm, the mammary gland region ratio Rm1, and the mammary gland mass ratio Rm2 may be transmitted from the breast composition information calculation unit 36 to the analysis determination unit 37.

The analysis determination unit 37 determines whether or not to perform analysis on the GTC region including mammary ducts, lobules, and peripheral stromata in the mammary gland region based on the breast composition information acquired by the breast composition information acquisition unit F.

In Su Hyun Lee et al. "Glandular Tissue Component and Breast Cancer Risk in Mammographically Dense Breasts at Screening Breast US", Radiology, Volume 301, October 1, 2021, it is reported that a ratio of the GTC region in the mammary gland region can be a cancer risk factor, and analysis on the GTC region in the mammary gland region has great significance in estimating a cancer risk. On the other hand, in a case where a ratio of a mammary gland region to a breast region is decreased due to retraction of mammary glands and a decrease in a volume of a breast, a cancer risk is reduced. Thus, a necessity to analyze the GTC region in the mammary gland region is reduced.

Further, it is difficult to distinguish a peripheral stroma and an edematous stroma in the mammography image obtained by the mammography apparatus. As a result, it is necessary to use an ultrasound image in order to analyze the GTC region.

Therefore, the analysis determination unit 37 determines whether or not to perform analysis on the GTC region based on the breast composition information of the subject that is obtained in the mammography examination. That is, in a case where it is determined that it is desired to estimate a cancer risk of the mammary gland region in more detail based on the breast composition information of the subject acquired by the breast composition information acquisition unit F, it is determined to perform analysis on the GTC region. On the other hand, in a case where it is determined that a cancer risk is low based on the breast composition information, it is determined not to perform analysis on the GTC region using the ultrasound image.

Thereby, it is possible to effectively estimate a cancer risk in accordance with the subject.

Specifically, in a case where the mammary gland category Cm of the subject is input from the main-body-side communication unit 35, the analysis determination unit 37 determines whether or not the input mammary gland category Cm is the mammary gland category C4 of "extremely high density" or the mammary gland category C3 of "heterogeneous high density" that is determined in order in which the mammary gland density is high among the mammary gland categories C1 to C4 classified into four types. In a case where the input mammary gland category Cm belongs to the mammary gland category C4 or C3 indicating a high mammary gland density, the analysis determination unit 37 determines to perform analysis on the GTC region. On the other hand, in a case where the input mammary gland category Cm is the mammary gland category C2 of "scattered mammary gland" or the mammary gland category C1 of "fatty", which indicates a relatively-low mammary gland density, the analysis determination unit 37 determines that a cancer risk is low and determines not to perform analysis on the GTC region.

The determination is not limited to performing analysis on the GTC region in a case where the mammary gland category Cm of the subject belongs to two mammary gland categories C4 and C3 that are determined in order in which the mammary gland density is high among the mammary gland categories C1 to C4 classified into four types. For example, in a case where the mammary gland category Cm of the subject is any one of the mammary gland category C4 of "extremely high density", the mammary gland category C3 of "heterogeneous high density", or the mammary gland category C2 of "scattered mammary gland", the mammary gland categories C4, C3, and C2 being determined in order in which the mammary gland density is high, the analysis determination unit 37 may determine to perform analysis on the GTC region. Further, in a case where the mammary gland category Cm of the subject is the mammary gland category C4 of "extremely high density" indicating the highest mammary gland density, the analysis determination unit 37 may determine to perform analysis on the GTC region.

In addition, in the BI-RADS 5th edition described above, classification of four types of mammary gland categories is recommended. On the other hand, the present invention is not limited thereto. In a case where the mammary gland category Cm of the subject belongs to at least one mammary gland category that is determined in order in which the mammary gland density is high among the mammary gland categories classified into a plurality of types, the analysis determination unit 37 can determine to perform analysis on the GTC region.

Further, in a case where the mammary gland region ratio Rm1 of the subject is input from the main-body-side communication unit 35, the analysis determination unit 37 compares the input mammary gland region ratio Rm1 with a predetermined mammary gland region threshold value Th1. In a case where the mammary gland region ratio Rm1 is higher than the mammary gland region threshold value Th1, the analysis determination unit 37 determines to perform analysis on the GTC region. In a case where the mammary gland region ratio Rm1 is equal to or lower than the mammary gland region threshold value Th1, the analysis determination unit 37 determines that a cancer risk is low and determines not to perform analysis on the GTC region.

Similarly, even in a case where the mammary gland region ratio Rm1 calculated by the breast composition information calculation unit 36 is input from the breast composition information calculation unit 36, the analysis determination unit 37 compares the input mammary gland region ratio Rm1 with a predetermined mammary gland region threshold value Th1. In a case where the mammary gland region ratio Rm1 is higher than the mammary gland region threshold value Th1, the analysis determination unit 37 determines to perform analysis on the GTC region. In a case where the mammary gland region ratio Rm1 is equal to or lower than the mammary gland region threshold value Th1, the analysis determination unit 37 determines not to perform analysis on the GTC region.

Further, in a case where the mammary gland mass ratio Rm2 of the subject is input from the main-body-side communication unit 35 or the breast composition information calculation unit 36, the analysis determination unit 37 compares the input mammary gland mass ratio Rm2 with a predetermined mammary gland mass threshold value Th2. In a case where the mammary gland mass ratio Rm2 is higher than the mammary gland mass threshold value Th2, the analysis determination unit 37 determines to perform analysis on the GTC region. In a case where the mammary gland mass ratio Rm2 is equal to or lower than the mammary gland mass threshold value Th2, the analysis determination unit 37 determines that a cancer risk is low and determines not to perform analysis on the GTC region.

In a case where the analysis determination unit 37 determines to perform analysis on the GTC region, the GTC region analysis unit 38 performs analysis on the GTC region based on an ultrasound image obtained by imaging the breast of the subject. As illustrated in Fig. 5, the GTC region analysis unit 38 includes a breast region detection unit 57 connected to the analysis determination unit 37 and the image memory 34, a mammary gland region detection unit 58 connected to the breast region detection unit 57, a GTC region extraction unit 59 connected to the mammary gland region detection unit 58, and a GTC region ratio calculation unit 60 connected to the mammary gland region detection unit 58 and the GTC region extraction unit 59. The GTC region extraction unit 59 and the GTC region ratio calculation unit 60 are connected to the display control unit 32.

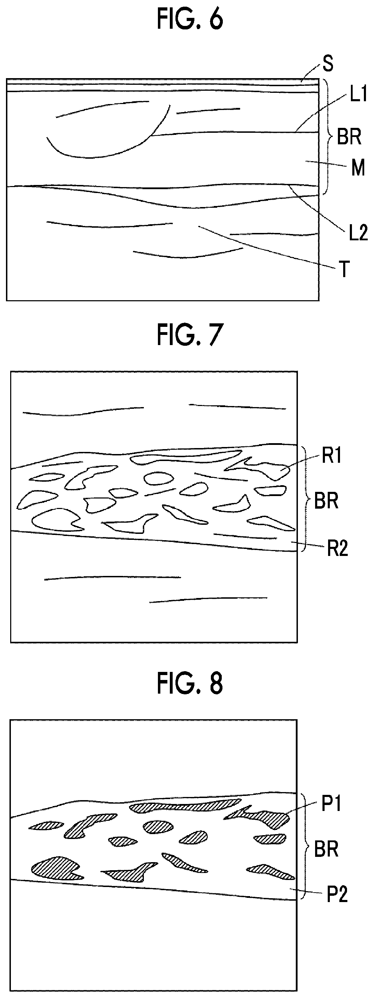

The breast region detection unit 57 detects a breast region of the subject from the ultrasound image generated by the image generation unit 31. Fig. 6 illustrates an example of an ultrasound image obtained by imaging a breast of a subject. The ultrasound image is a tomographic image obtained by bringing a tip of the ultrasound probe 2 into contact with the breast of the subject and performing imaging. A skin S of the subject appears in an upper end of the ultrasound image representing the shallowest portion, and a pectoralis major muscle T appears in a lower portion of the ultrasound image representing a deeper portion. The breast region detection unit 57 can recognize the skin S and the pectoralis major muscle T from the ultrasound image, and detect a deep region between the skin S and the pectoralis major muscle T, as a breast region BR.

The mammary gland region detection unit 58 detects a mammary gland region of the subject from the ultrasound image generated by the image generation unit 31. As illustrated in Fig. 6, the mammary gland region detection unit 58 can recognize a front boundary line L1 located on a shallower side and a rear boundary line L2 located on a deeper side in the breast region BR detected by the breast region detection unit 57, and detect a deep region between the front boundary line L1 and the rear boundary line L2, as a mammary gland region M.

In order to detect the breast region BR and the mammary gland region M described above, image recognition can be performed using at least one of template matching, an image analysis technique using feature amounts such as adaptive boosting (Adaboost), support vector machine (SVM), or scale-invariant feature transform (SIFT), or a determination model learned by using a machine learning technique such as deep learning.

Note that the determination model is a learned model obtained by learning the breast region BR and the mammary gland region M (segmentation) of the breast region BR in a learning ultrasound image obtained by imaging the breast.

The GTC region extraction unit 59 extracts a GTC region from the mammary gland region M of the subject that is detected by the mammary gland region detection unit 58. The GTC region includes mammary ducts, lobules, and peripheral stromata in the mammary gland region M, and an edematous stroma fills a space between the peripheral stromata. In the edematous stroma, substrates are rich and adipocytes coexist. For this reason, in a case where the mammary gland region M is observed in an ultrasound image, the edematous stroma has a high echo level and appears with high brightness. On the other hand, the mammary ducts, the lobules, and the peripheral stromata that are included in the GTC region have relatively low echo levels, and have brightness lower than the brightness of the edematous stroma.

For this reason, the GTC region extraction unit 59 binarizes the mammary gland region M of the ultrasound image by using, for example, a brightness threshold value Thb. Thus, the GTC region and the edematous stroma in the mammary gland region M are distinguished from each other. Thereby, the GTC region can be extracted.

For example, as in the ultrasound image illustrated in Fig. 7, in a case where the GTC region R1 and the edematous region R2 filled with the edematous stroma coexist in the mammary gland region M, by binarizing the mammary gland region M by using an appropriate brightness threshold value Thb, a binarized image as illustrated in Fig. 8 is obtained.

In the binarized image of Fig. 8, pixels having brightness values lower than the brightness threshold value Thb are represented in black (a shaded regions in Fig. 8), and the pixels form a black portion P1. In addition, pixels having brightness values equal to or higher than the brightness threshold value Thb are represented in white, and the pixels form a white portion P2. The black portion P1 corresponds to the GTC region R1, and the white portion P2 corresponds to the edematous region R2. That is, by binarizing the mammary gland region M, the GTC region R1 can be extracted as the black portion P1.

The binarized image created by the GTC region extraction unit 59 is displayed on the monitor 33 via the display control unit 32.

Note that a predetermined constant value can be used as the brightness threshold value Thb.

In addition, the GTC region extraction unit 59 may perform edge detection on the GTC region R1 of the ultrasound image by image analysis, and automatically calculate a brightness threshold value Thb based on a change in brightness values of a detected edge portion, that is, a change in brightness values of a plurality of pixels from the inside to the outside of the GTC region R1. In this way, it is possible to automatically set the brightness threshold value Thb suitable for the ultrasound image as an image analysis target, and to acquire a binarized image suitable for the ultrasound image.

Further, in a case where a histogram of the brightness of the mammary gland region M detected from the ultrasound image is created, it is possible to allow the user to input and set the brightness threshold value Thb via the input device 41 based on the histogram, the binarized image created by using an initial value of the brightness threshold value Thb, and the ultrasound image generated by the image generation unit 31.

In addition, the GTC region extraction unit 59 can also extract the GTC region R1 by using a determination model which is learned using a machine learning technique such as deep learning. In this case, a learned model obtained by learning the GTC region R1 (segmentation) of the mammary gland region M in the learning ultrasound image obtained by imaging the breast is used as the determination model.

The GTC region ratio calculation unit 60 calculates a ratio of the GTC region R1 to the mammary gland region M, and transmits the calculated ratio to the display control unit 32. Specifically, the GTC region ratio calculation unit 60 receives the mammary gland region M detected by the mammary gland region detection unit 58 and the GTC region R1 extracted by the GTC region extraction unit 59, and calculates a GTC region ratio of the GTC region R1 to the mammary gland region M.

In addition, the GTC region ratio calculation unit 60 stores the calculated GTC region ratio in the examination result memory 39, as an examination result.

The GTC region ratio cannot be measured by a mammography apparatus. On the other hand, in the ultrasound diagnostic apparatus according to the embodiment, the GTC region ratio can be calculated based on, for example, the number of occupied pixels of the mammary gland region M and the number of occupied pixels of the GTC region R1 in the ultrasound image. Specifically, the GTC region ratio is represented by a ratio of a sum of the number of occupied pixels of all the GTC regions R1 existing in the mammary gland region M to the number of occupied pixels of the entire mammary gland region M.

The GTC region ratio calculation unit 60 may display the calculated GTC region ratio on the monitor 33 by using a numerical value, or may display the calculated GTC region ratio on the monitor 33 by using a pie graph, a bar graph, or the like.

The examination result memory 39 is a memory that stores, as an examination result, the GTC region ratio which is calculated by the GTC region ratio calculation unit 60 of the GTC region analysis unit 38 under a control of the main body control unit 40.

Similar to the image memory 34, as the examination result memory 39, a recording medium such as a flash memory, an HDD, an SSD, an FD, an MO disc, an MT, a RAM, a CD, a DVD, an SD card, a USB memory, or the like can be used.

Note that the examination result memory 39 may be either a memory integrated with the image memory 34 or a memory separated from the image memory 34.

In addition, the ultrasound image generated by the image generation unit 31 is represented, for example, as image data with a so-called digital imaging and communications in medicine (DICOM) format in which patient identification information is added. The ultrasound image includes a tag for storing accessory information, and the GTC region ratio which is calculated by the GTC region ratio calculation unit 60 of the GTC region analysis unit 38 can be stored with a tag associated with the ultrasound image.

The main body control unit 40 controls each unit of the diagnostic apparatus main body 3 and the transmission/reception circuit 22 of the ultrasound probe 2 based on a control program or the like which is stored in advance.

In addition, a main-body-side storage unit (not illustrated) is connected to the main body control unit 40. The main-body-side storage unit stores a control program or the like. In addition, as the main-body-side storage unit, for example, a flash memory, a RAM, an SD card, an SSD, or the like can be used.

The input device 41 is a device that allows a user to perform an input operation, and includes, for example, devices such as a keyboard, a mouse, a trackball, a touch pad, and a touch sensor overlapped and provided on the monitor 33.

Note that the processor 42 including the image generation unit 31, the display control unit 32, the main-body-side communication unit 35, the breast composition information calculation unit 36, the analysis determination unit 37, the GTC region analysis unit 38, and the main body control unit 40 is configured with a central processing unit (CPU) and a control program for causing the CPU to perform various processing. The processor 42 may be configured by using a field programmable gate array (FPGA), a digital signal processor (DSP), an application specific integrated circuit (ASIC), a graphics processing unit (GPU), or other integrated circuits (IC), or may be configured by using a combination thereof.

In addition, the image generation unit 31, the display control unit 32, the main-body-side communication unit 35, the breast composition information calculation unit 36, the analysis determination unit 37, the GTC region analysis unit 38, and the main body control unit 40 of the processor 42 can be partially or entirely integrated into one CPU or the like.

The ultrasound image analysis apparatus 4 that includes the main-body-side communication unit 35, the breast composition information calculation unit 36, the analysis determination unit 37, the GTC region analysis unit 38, and the main body control unit 40 of the processor 42 may be configured with an FPGA, a DSP, an ASIC, a GPU, or other ICs independently from the image generation unit 31 and the display control unit 32.

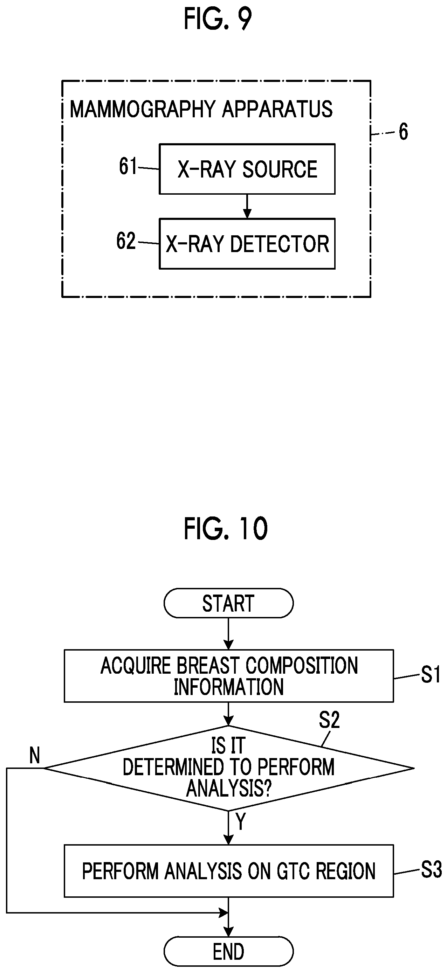

As illustrated in Fig. 9, the mammography apparatus 6 connected to the diagnostic apparatus main body 3 via the network 5 includes an X-ray source 61 and an X-ray detector 62.

For example, the breast of the subject compressed by a compression plate (not illustrated) is irradiated with an X-ray from the X-ray source 61 while changing an incidence angle of the X-ray, and the X-ray passing through the breast is detected by the X-ray detector 62. Thereby, a mammography image is acquired.

In addition, the mammography apparatus 6 includes a computer (not illustrated), and the computer can calculate a mammary gland region ratio Rm1 which is a ratio of a mammary gland region to a breast region of a subject or a mammary gland mass ratio Rm2 which is a ratio of a mammary gland mass to a mammary gland region, from the mammography image.

Further, the mammography apparatus 6 can also calculate the mammary gland category Cm of the subject based on the mammary gland region ratio Rm1 or the mammary gland mass ratio Rm2.

The server 7 connected to the diagnostic apparatus main body 3 via the network 5 stores various image data generated by the ultrasound diagnostic apparatus 1 and the mammography apparatus 6. Specifically, the ultrasound image generated by the diagnostic apparatus main body 3 and the mammography image generated by the mammography apparatus 6 can be stored in the server 7.

The server 7 can be used as a server in a so-called picture archiving and communication system (PACS, a medical image management system) or a workstation.

In addition, a computer included in the server 7 can calculate a mammary gland region ratio Rm1 which is a ratio of a mammary gland region to a breast region of a subject or a mammary gland mass ratio Rm2 which is a ratio of a mammary gland mass to a mammary gland region, from the mammography image.

Further, the server 7 can also store the mammary gland category Cm of the subject that is obtained by a mammography examination in the mammography apparatus 6.

Next, an operation of the ultrasound diagnostic apparatus 1 according to the embodiment will be described with reference to a flowchart illustrated in Fig. 10.

First, in step S1, breast composition information, which is information related to breast composition of the subject obtained by the mammography examination, is acquired by the breast composition information acquisition unit F of the diagnostic apparatus main body 3.

Specifically, as the breast composition information, the mammary gland category Cm, the mammary gland region ratio Rm1, or the mammary gland mass ratio Rm2 of the subject, which is transmitted from the mammography apparatus 6 or the server 7 to the main-body-side communication unit 35 via the network 5, is acquired. In addition, the breast composition information calculation unit 36 may calculate a mammary gland region ratio Rm1 of the subject based on the mammography image transmitted from the mammography apparatus 6 or the server 7 to the main-body-side communication unit 35 via the network 5, and may acquire the calculated mammary gland region ratio Rm1 as the breast composition information. Further, the mammary gland category Cm and the mammary gland mass ratio Rm2 that are calculated by the breast composition information calculation unit 36 may be acquired as the breast composition information.

Next, in step S2, the analysis determination unit 37 performs analysis determination processing of determining whether or not to perform analysis on the GTC region based on the breast composition information acquired in step S1.

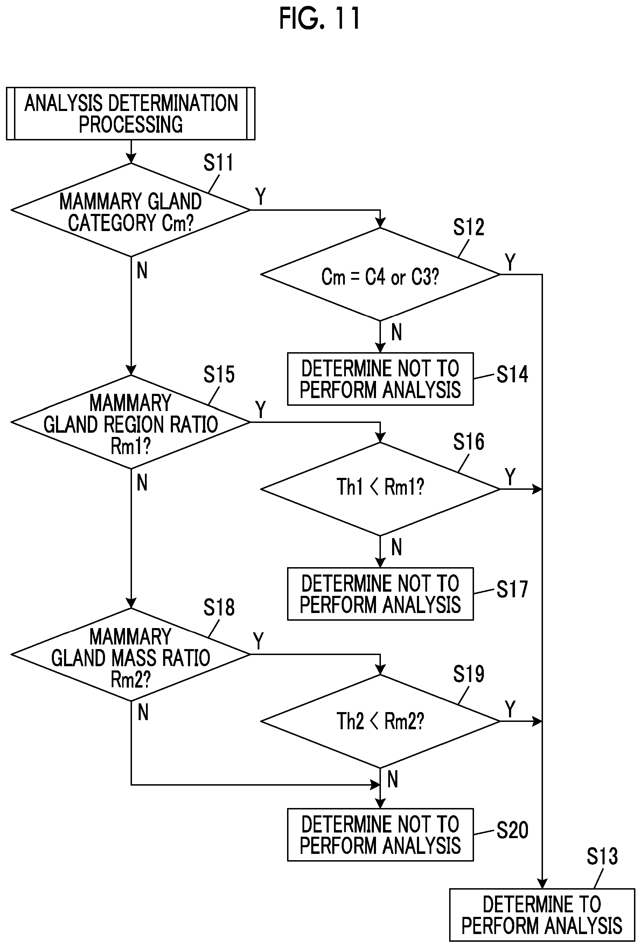

An operation of the analysis determination processing in the analysis determination unit 37 is illustrated in a flowchart of Fig. 11.

In step S11, it is determined whether or not the breast composition information which is input from the breast composition information acquisition unit F to the analysis determination unit 37 is the mammary gland category Cm of the subject. In step S11, in a case where it is determined that the breast composition information is the mammary gland category Cm of the subject, the processing proceeds to step S12. Further, it is determined whether or not the mammary gland category Cm of the subject belongs to the mammary gland category C4 of "extremely high density" or the mammary gland category C3 of "heterogeneous high density" that is determined in order in which the mammary gland density is high among the mammary gland categories C1 to C4 classified into four types.

In step S12, in a case where it is determined that the mammary gland category Cm of the subject is the mammary gland category C4 of "extremely high density" or the mammary gland category C3 of "heterogeneous high density", it is determined that it is necessary to estimate a cancer risk by analyzing the GTC region, and the processing proceeds to step S13. Then, it is determined to perform analysis on the GTC region. On the other hand, in step S12, in a case where it is determined that the mammary gland category Cm of the subject is neither the mammary gland category C4 of "extremely high density" nor the mammary gland category C3 of "heterogeneous high density", it is determined that a cancer risk is low, and the processing proceeds to step S14. Then, it is determined not to perform analysis on the GTC region.

In a case where it is determined in step S11 that the breast composition information which is input from the breast composition information acquisition unit F to the analysis determination unit 37 is not the mammary gland category Cm of the subject, in step S15, it is determined whether or not the breast composition information which is input from the breast composition information acquisition unit F to the analysis determination unit 37 is the mammary gland region ratio Rm 1.

In a case where it is determined in step S15 that the mammary gland region ratio Rm1 of the subject is input, the processing proceeds to step S16, and the input mammary gland region ratio Rm1 is compared with the predetermined mammary gland region threshold value Th1. In a case where it is determined that the mammary gland region ratio Rm1 is higher than the mammary gland region threshold value Th1, it is determined that it is necessary to estimate a cancer risk by analyzing the GTC region, and the processing proceeds to step S13. Then, it is determined to perform analysis on the GTC region. On the other hand, in a case where it is determined in step S16 that the mammary gland region ratio Rm1 of the subject is equal to or lower than the predetermined mammary gland region threshold value Th1, it is determined that a cancer risk is low, and the processing proceeds to step S17. Then, it is determined not to perform analysis on the GTC region.

Further, in a case where it is determined in step S15 that the breast composition information which is input from the breast composition information acquisition unit F to the analysis determination unit 37 is not the mammary gland region ratio Rm1, in step S18, it is determined whether or not the breast composition information which is input from the breast composition information acquisition unit F to the analysis determination unit 37 is the mammary gland mass ratio Rm2.

In a case where it is determined in step S18 that the mammary gland mass ratio Rm2 of the subject is input, the processing proceeds to step S19, and the input mammary gland mass ratio Rm2 is compared with the predetermined mammary gland mass threshold value Th2. In a case where it is determined that the mammary gland mass ratio Rm2 is higher than the mammary gland mass threshold value Th2, it is determined that it is necessary to estimate a cancer risk by analyzing the GTC region, and the processing proceeds to step S13. Then, it is determined to perform analysis on the GTC region. On the other hand, in a case where it is determined in step S19 that the mammary gland mass ratio Rm2 of the subject is equal to or lower than the predetermined mammary gland mass threshold value Th2, it is determined that a cancer risk is low, and the processing proceeds to step S20. Then, it is determined not to perform analysis on the GTC region.

In addition, in a case where it is determined in step S18 that the breast composition information which is input from the breast composition information acquisition unit F to the analysis determination unit 37 is not the mammary gland mass ratio Rm2, it is determined that the breast composition information which is useful to determine whether to perform analysis on the GTC region is not acquired, and the processing proceeds to step S20. Then, it is determined not to perform analysis on the GTC region.

In this way, in step S2 of Fig. 10, the analysis determination unit 37 determines whether or not to perform analysis on the GTC region.

In step S2, in a case where it is determined to perform analysis on the GTC region, the processing proceeds to step S3. Then, the GTC region analysis unit 38 performs analysis processing on the GTC region based on the ultrasound image obtained by imaging the breast of the subject.

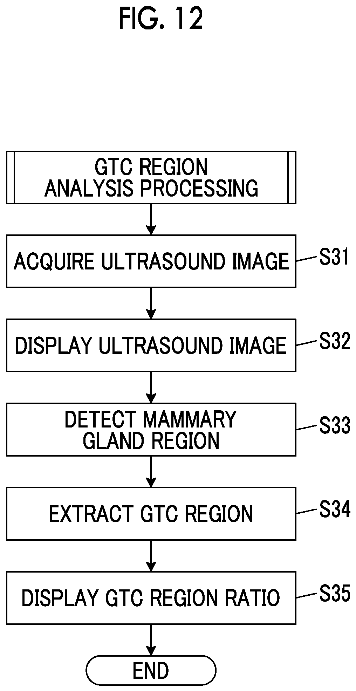

An operation of the GTC region analysis processing in the GTC region analysis unit 38 is illustrated in a flowchart of Fig. 12.

First, in step S31, a breast of a subject is imaged by using the ultrasound probe 2, and thus an ultrasound image is acquired. At this time, under a control of the main body control unit 40, transmission and reception of ultrasound waves from the plurality of transducers of the transducer array 21 are started according to a drive signal from the pulser 23 of the transmission/reception circuit 22 of the ultrasound probe 2. The ultrasound echo from the inside of the breast of the subject is received by the plurality of transducers of the transducer array 21. The reception signal which is an analog signal is output to the amplification unit 24, and is amplified by the amplification unit 24. The amplified reception signal is AD-converted by the AD conversion unit 25, and thus the reception data is acquired.

The reception focus processing is performed on the reception data by the beam former 26, and the sound ray signal generated by the reception focus processing is transmitted to the image generation unit 31 of the diagnostic apparatus main body 3. An ultrasound image representing tomographic image information of the breast of the subject is generated by the image generation unit 31. At this time, attenuation correction according to a depth of a reflection position of the ultrasound wave and envelope detection processing are performed on the sound ray signal by the signal processing unit 51 of the image generation unit 31. The sound ray signal is converted into an image signal conforming to a scanning method of a normal television signal by the DSC 52, and various required image processing such as gradation processing is performed on the image signal by the image processing unit 53.

Subsequently, in step S32, the ultrasound image generated by the image generation unit 31 is displayed on the monitor 33 via the display control unit 32, and is stored in the image memory 34.

Note that, in a case where an ultrasound image is acquired, under a control of the main body control unit 40, a transmission intensity of the ultrasound wave and a depth range of the ultrasound image displayed on the monitor 33 are adjusted such that the entire breast of the subject, that is, for example, the deep portion between the skin S and the pectoralis major muscle T of the subject illustrated in Fig. 6, is fitted into the screen.

In a case where the ultrasound image is stored in the image memory 34 in this way, in step S33, the ultrasound image is input to the GTC region analysis unit 38, and a breast region BR of the subject is detected from the ultrasound image by the breast region detection unit 57 illustrated in Fig. 5. For example, as illustrated in Fig. 6, the deep region between the skin S of the subject appearing in the shallowest portion of the ultrasound image and the pectoralis major muscle T appearing in the deeper portion is detected as the breast region BR.

Further, the ultrasound image is input to the mammary gland region detection unit 58 via the breast region detection unit 57. The mammary gland region detection unit 58 recognizes the front boundary line L1 and the rear boundary line L2 in the breast region BR detected by the breast region detection unit 57, and detects, as the mammary gland region M, the deep region between the front boundary line L1 and the rear boundary line L2.

Next, in step S34, the GTC region R1 is extracted from the mammary gland region M by the GTC region extraction unit 59 of the GTC region analysis unit 38 illustrated in Fig. 5, the mammary gland region M being detected by the mammary gland region detection unit 58. At this time, the GTC region extraction unit 59 binarizes the mammary gland region M of the ultrasound image illustrated in Fig. 7 by using, for example, a brightness threshold value Thb as a predetermined constant value, and thus a binarized image as illustrated in Fig. 8 is acquired. In the binarized image of Fig. 8, the black portion P1 corresponds to the extracted GTC region R1, and the white portion P2 corresponds to the edematous region R2 filled with the edematous stroma.

The binarized image created by the GTC region extraction unit 59 is displayed on the monitor 33 via the display control unit 32.

Subsequently, in step S35, the GTC region ratio of the GTC region R1 to the mammary gland region M is calculated by the GTC region ratio calculation unit 60 of the GTC region analysis unit 38, and the GTC region ratio is displayed on the monitor 33 via the display control unit 32. At this time, the GTC region ratio calculation unit 60 can calculate the GTC region ratio based on a ratio of the number of occupied pixels of all the GTC regions R1 existing in the mammary gland region M to the number of occupied pixels of the entire mammary gland region M in the ultrasound image.

The calculated GTC region ratio is displayed on the monitor 33, as a numerical value, a pie graph, a bar graph, or the like, and is also stored in the examination result memory 39, as an examination result. Further, the calculated GTC region ratio can be stored in a tag with a DICOM format by being associated with the ultrasound image, or can be directly transmitted from the ultrasound diagnostic apparatus 1 to an external server 7, a diagnostic report system, or the like via a network.

In this way, the GTC region ratio that cannot be measured by the mammography apparatus is displayed on the monitor 33.

By checking the GTC region ratio displayed on the monitor 33, it is possible to estimate a cancer risk of the mammary gland region of the subject.

In a case where a plurality of pieces of breast composition information including a mammary gland category Cm, a mammary gland region ratio Rm1, or a mammary gland mass ratio Rm2 are acquired by the breast composition information acquisition unit F, the analysis determination unit 37 determines to perform analysis on the GTC region based on each of the plurality of pieces of acquired breast composition information. In a case where a determination result indicating that it is determined to perform analysis on the GTC region is obtained from at least one piece of breast composition information among the plurality of pieces of breast composition information, it is possible to determine to perform analysis on the GTC region.

Alternatively, in a case where a result indicating that it is determined to perform analysis on the GTC region is obtained from all of the plurality of pieces of breast composition information acquired by the breast composition information acquisition unit F, it may be determined to perform analysis on the GTC region.

In the embodiment described above, the ultrasound image generated by the image generation unit 31 is a two-dimensional ultrasound image. On the other hand, the image generation unit 31 can also generate a three-dimensional ultrasound image of the breast of the subject. After acquiring a plurality of two-dimensional ultrasound images on a plurality of different tomographic planes by performing planar scanning on the subject by using the ultrasound probe 2, a three-dimensional ultrasound image may be generated based on the plurality of two-dimensional ultrasound images. Alternatively, by using a three-dimensional probe instead of the ultrasound probe 2, a three-dimensional ultrasound image may be generated in a state where the three-dimensional probe is stationary.

In this case, the mammary gland region M is detected from the three-dimensional ultrasound image by the mammary gland region detection unit 58 of the GTC region analysis unit 38, and the GTC region R1 is extracted from the mammary gland region M of the three-dimensional ultrasound image by the GTC region extraction unit 59. The GTC region ratio calculation unit 60 can calculate a GTC region ratio of the GTC region R1 to the mammary gland region M based on a volume of the mammary gland region M detected by the mammary gland region detection unit 58 and a volume of the GTC region R1 extracted by the GTC region extraction unit 59. The calculated GTC region ratio is displayed on the monitor 33, and is stored in the examination result memory 39.

By calculating the GTC region ratio based on the three-dimensional ultrasound image in this way, it is possible to improve accuracy of estimation of a cancer risk of the mammary gland region M of the subject, and perform diagnosis with higher reliability.

In the embodiment, the breast composition information calculation unit 36 of the breast composition information acquisition unit F calculates, as the breast composition information, the mammary gland region ratio Rm1 which is a ratio of the mammary gland region to the breast region of the subject, from the mammography image. On the other hand, the present invention is not limited thereto. The breast composition information calculation unit 36 may calculate, as the breast composition information, the mammary gland mass ratio Rm2 which is a ratio of the mammary gland mass to the mammary gland region of the subject and the mammary gland category Cm, from the mammography image.

In the embodiment described above, the ultrasound image analysis apparatus 4 is provided inside the ultrasound diagnostic apparatus 1. On the other hand, the present invention is not limited thereto. The ultrasound image analysis apparatus 4 can be used separately from the ultrasound diagnostic apparatus 1. For example, the ultrasound image analysis apparatus 4 may be provided inside the server 7 illustrated in Fig. 1 that is connected to a general-purpose ultrasound imaging apparatus via the network 5, an ultrasound image obtained by imaging the breast of the subject may be transmitted to the server 7 by the general-purpose ultrasound imaging apparatus, and the GTC region ratio may be calculated by the ultrasound image analysis apparatus 4. Further, for example, as described in JP2008-161283A, JP2019-69319A, and the like, the ultrasound image analysis apparatus 4 may be provided in a mammography apparatus having an ultrasound measurement function, and the GTC region ratio can be calculated inside the mammography apparatus.

The method of connecting the ultrasound probe 2 and the diagnostic apparatus main body 3 in the embodiment is not particularly limited, and a wired connection method or a wireless connection method may be used.

In the embodiment, the ultrasound probe 2 includes the transmission/reception circuit 22. On the other hand, the diagnostic apparatus main body 3 may include the transmission/reception circuit 22. In addition, the diagnostic apparatus main body 3 includes the image generation unit 31. On the other hand, the ultrasound probe 2 may include the image generation unit 31. Further, the image generation unit 31 illustrated in Fig. 3 includes the signal processing unit 51, the DSC 52, and the image processing unit 53. On the other hand, the ultrasound probe 2 may include only the signal processing unit 51, and the diagnostic apparatus main body 3 may include the DSC 52 and the image processing unit 53.

In addition, in the embodiment, as the diagnostic apparatus main body 3, a portable or handheld compact diagnostic apparatus main body can be used, and a stationary diagnostic apparatus main body can also be used.

### Explanation of References

1: ultrasound diagnostic apparatus
2: ultrasound probe
3: diagnostic apparatus main body
4: ultrasound image analysis apparatus
5: network
6: mammography apparatus
7: server
21: transducer array
22: transmission/reception circuit
23: pulser
24: amplification unit
25: AD conversion unit
26: beam former
31: image generation unit
32: display control unit
33: monitor
34: image memory
35: main-body-side communication unit
36: breast composition information calculation unit
37: analysis determination unit
38: GTC region analysis unit
39: examination result memory
40: main body control unit
41: input device
42: processor
51: signal processing unit
52: DSC
53: image processing unit
54, 57: breast region detection unit
55, 58: mammary gland region detection unit
56: mammary gland region ratio calculation unit
59: GTC region extraction unit
60: GTC region ratio calculation unit
61: X-ray source
62: X-ray detector
S: skin
BR: breast region
M: mammary gland region
L1: front boundary line
L2: rear boundary line
T: pectoralis major muscle
R1: GTC region
R2: edematous region
P1: black portion
P2: white portion

## Claims

1. An ultrasound image analysis apparatus comprising:
a breast composition information acquisition unit that is configured to acquire information related to a breast composition of a subject obtained by a mammography examination;
an analysis determination unit (37) that is configured to determine whether or not to perform analysis on a glandular tissue component region including mammary ducts, lobules, and peripheral stromata in a mammary gland region based on the information related to the breast composition acquired by the breast composition information acquisition unit (36); and
a glandular tissue component region analysis unit (38) that is configured to perform analysis on the glandular tissue component region based on an ultrasound image obtained by imaging a breast of the subject in a case where the analysis determination unit (37) determines to perform analysis;
**characterised in that**
the glandular tissue component region analysis unit (38) is configured to calculate a ratio of the glandular tissue component region to the mammary gland region; and
**in that** the glandular tissue component region analysis unit (38) includes:
a mammary gland region detection unit (55, 58) that is configured to detect the mammary gland region from the ultrasound image;
a glandular tissue component region extraction unit (59) that is configured to extract the glandular tissue component region in the mammary gland region detected by the mammary gland region detection unit (55, 58); and
a glandular tissue component region ratio calculation unit (60) that is configured to calculate the ratio of the glandular tissue component region to the mammary gland region.

2. The ultrasound image analysis apparatus according to claim 1,
wherein the breast composition information acquisition unit is configured to acquire, as the information related to the breast composition, a mammary gland category corresponding to the subject among mammary gland categories classified into a plurality of types according to a mammary gland density.

3. The ultrasound image analysis apparatus according to claim 2,
wherein the analysis determination unit (37) is configured to determine to perform analysis on the glandular tissue component region in a case where the mammary gland category corresponding to the subject belongs to at least one mammary gland category which is determined in order in which the mammary gland density is high among the mammary gland categories classified into the plurality of types.

4. The ultrasound image analysis apparatus according to claim 1,
wherein the breast composition information acquisition unit is configured to acquire, as the information related to the breast composition, a ratio of the mammary gland region to a breast region of the subject.

5. The ultrasound image analysis apparatus according to claim 4,
wherein the breast composition information acquisition unit includes a breast composition information calculation unit (36) that is configured to calculate the ratio of the mammary gland region to the breast region of the subject from a mammography image.

6. The ultrasound image analysis apparatus according to claim 4 or 5,
wherein the analysis determination unit (37) is configured to determine to perform analysis on the glandular tissue component region in a case where the ratio of the mammary gland region to the breast region is higher than a predetermined mammary gland region threshold value.

7. The ultrasound image analysis apparatus according to claim 1,
wherein the breast composition information acquisition unit is configured to acquire, as the information related to the breast composition, a ratio of a mammary gland mass to the mammary gland region of the subject.

8. The ultrasound image analysis apparatus according to claim 7,
wherein the analysis determination unit (37) is configured to determine to perform analysis on the glandular tissue component region in a case where the ratio of the mammary gland mass to the mammary gland region is higher than a predetermined mammary gland mass threshold value.

9. The ultrasound image analysis apparatus according to any one of claims 1-8,
wherein the mammary gland region detection unit (55, 58) is configured to detect the mammary gland region by image recognition of the ultrasound image.

10. The ultrasound image analysis apparatus according to claim 9, further comprising:
a breast region detection unit (54, 57) that is configured to detect a breast region located between a skin and a pectoralis major muscle from the ultrasound image,
wherein the mammary gland region detection unit (55, 58) is configured to recognize a front boundary line and a rear boundary line in the breast region detected by the breast region detection unit (54, 57), and detect a region between the front boundary line and the rear boundary line, as the mammary gland region.

11. The ultrasound image analysis apparatus according to any one of claims 1-8,
wherein the mammary gland region detection unit (55, 58) is configured to detect the mammary gland region from the ultrasound image by using deep learning.

12. The ultrasound image analysis apparatus according to any one of claims 1 to 11,
wherein the glandular tissue component region extraction unit (59) is configured to extract the glandular tissue component region by binarizing the mammary gland region of the ultrasound image by using a brightness threshold value.

13. The ultrasound image analysis apparatus according to any one of claims 1 to 11,
wherein the glandular tissue component region extraction unit (59) is configured to extract the glandular tissue component region from the mammary gland region of the ultrasound image by using deep learning.

14. The ultrasound image analysis apparatus according to any one of claims 1 to 13,
wherein the glandular tissue component region ratio calculation unit (60) is configured to calculate the ratio of the glandular tissue component region to the mammary gland region based on the number of occupied pixels of the mammary gland region and the number of occupied pixels of the glandular tissue component region in the ultrasound image.

15. The ultrasound image analysis apparatus according to any one of claims 1 to 14,
wherein the ultrasound image is a three-dimensional ultrasound image, and
the glandular tissue component region ratio calculation unit (60) is configured to calculate the ratio of the glandular tissue component region to the mammary gland region based on a volume of the mammary gland region detected by the mammary gland region detection unit (55, 58) and a volume of the glandular tissue component region extracted by the glandular tissue component region extraction unit (59).

16. An ultrasound diagnostic apparatus comprising:
a monitor (33) that is configured to display an ultrasound image; and
the ultrasound image analysis apparatus (4) according to any one of claims 1 to 15,
wherein the glandular tissue component region analysis unit (38) is configured to display an analysis result of the glandular tissue component region on the monitor (33).

17. The ultrasound diagnostic apparatus according to claim 16,
wherein the glandular tissue component region analysis unit (38) is configured to store the analysis result of the glandular tissue component region in a tag associated with the ultrasound image.

18. The ultrasound diagnostic apparatus according to claim 16 or 17, further comprising:
an ultrasound probe (2); and
an image generation unit (31) that is configured to generate the ultrasound image obtained by imaging the breast of the subject by transmitting and receiving an ultrasound beam to and from the subject using the ultrasound probe (2).

19. A computer implemented control method for an ultrasound image analysis apparatus, the method comprising:
acquiring information related to a breast composition of a subject obtained by a mammography examination;
determining whether or not to perform analysis on a glandular tissue component region including mammary ducts, lobules, and peripheral stromata in a mammary gland region based on the acquired information related to the breast composition; **characterised in**
performing analysis on the glandular tissue component region based on an ultrasound image obtained by imaging a breast of the subject in a case where it is determined to perform analysis;
wherein the analysis on the glandular tissue component region is performed by:
detecting the mammary gland region from the ultrasound image;
extracting the glandular tissue component region in the mammary gland region detected; and
calculating the ratio of the glandular tissue component region to the mammary gland region.

## Patentansprüche

1. Ultraschallbild-Analysevorrichtung, umfassend:
eine Brustzusammensetzungsinformations-Erfassungseinheit, die so konfiguriert ist, dass sie Informationen, die sich auf eine Brustzusammensetzung einer Untersuchungsperson beziehen und durch eine Mammographieuntersuchung erhalten wurden, erfasst;
eine Analysebestimmungseinheit (37), die so konfiguriert ist, dass sie auf der Grundlage der Informationen, die sich auf die Brustzusammensetzung beziehen und von der Brustzusammensetzungsinformations-Erfassungseinheit (36) erfasst wurden, bestimmt, ob Analyse an einem Drüsengewebskomponentenbereich, der Milchgänge, Läppchen und peripheres Stroma enthält, in einem Brustdrüsenbereich durchgeführt werden soll oder nicht; und
eine Drüsengewebskomponentenbereichs-Analyseeinheit (38), die so konfiguriert ist, dass sie Analyse an dem Drüsengewebskomponentenbereich auf der Grundlage eines Ultraschallbildes, das durch Abbilden einer Brust der Untersuchungsperson erhalten wird, in einem Fall, in dem die Analysebestimmungseinheit (37) bestimmt, Analyse durchzuführen, durchführt;
**dadurch gekennzeichnet, dass**
die Drüsengewebskomponentenbereichs-Analyseeinheit (38) so konfiguriert ist, dass sie ein Verhältnis des Drüsengewebskomponentenbereichs zu dem Brustdrüsenbereich berechnet; und
dadurch, dass
die Drüsengewebskomponentenbereichs-Analyseeinheit (38) enthält:
eine Brustdrüsenbereich-Detektionseinheit (55, 58), die so konfiguriert ist, dass sie den Brustdrüsenbereich aus dem Ultraschallbild detektiert;
eine Drüsengewebskomponentenbereichs-Extraktionseinheit (59), die so konfiguriert ist, dass sie den Drüsengewebekomponentenbereich in dem Brustdrüsenbereich, der von der Brustdrüsenbereichs-Detektionseinheit (55, 58) detektiert wurde, extrahiert; und
eine Drüsengewebskomponentenbereichs-Verhältnis-Berechnungseinheit (60), die so konfiguriert ist, dass sie das Verhältnis des Drüsengewebskomponentenbereichs zu dem Brustdrüsenbereich berechnet.

2. Ultraschallbild-Analysevorrichtung nach Anspruch 1,
wobei die Brustzusammensetzungsinformations-Erfassungseinheit so konfiguriert ist, dass sie als die Informationen, die sich auf die Brustzusammensetzung beziehen, eine Brustdrüsenkategorie, die der Untersuchungsperson entspricht, unter Brustdrüsenkategorien, die gemäß einer Brustdrüsendichte in mehrere Typen klassifiziert sind, erfasst.

3. Ultraschallbild-Analysevorrichtung nach Anspruch 2,
wobei die Analysebestimmungseinheit (37) so konfiguriert ist, dass sie in einem Fall, in dem die Brustdrüsenkategorie, die der Untersuchungsperson entspricht, zu mindestens einer Brustdrüsenkategorie, die in Reihenfolge bestimmt wird, in der die Brustdrüsendichte hoch ist, unter den Brustdrüsenkategorien, die in die mehreren Typen klassifiziert sind, gehört, bestimmt, eine Analyse des Drüsengewebekomponentenbereichs durchzuführen.

4. Ultraschallbild-Analysevorrichtung nach Anspruch 1,
wobei die Brustzusammensetzungsinformations-Erfassungseinheit so konfiguriert ist, dass sie als die Informationen, die sich auf die Brustzusammensetzung beziehen, ein Verhältnis des Brustdrüsenbereichs zu einem Brustbereich der Untersuchungsperson erfasst.

5. Ultraschallbild-Analysevorrichtung nach Anspruch 4,
wobei die Brustzusammensetzungsinformations-Erfassungseinheit eine Brustzusammensetzungsinformations-Berechnungseinheit (36) enthält, die so konfiguriert ist, dass sie das Verhältnis des Brustdrüsenbereichs zu dem Brustbereich der Untersuchungsperson aus einem Mammographiebild berechnet.

6. Ultraschallbild-Analysevorrichtung nach Anspruch 4 oder 5,
wobei die Analysebestimmungseinheit (37) so konfiguriert ist, dass sie in einem Fall, in dem das Verhältnis des Brustdrüsenbereichs zu dem Brustbereich höher als ein vorbestimmter Brustdrüsenbereichs-Schwellenwert ist, bestimmt, Analyse an dem Drüsengewebskomponentenbereich durchzuführen.

7. Ultraschallbild-Analysevorrichtung nach Anspruch 1,
wobei die Brustzusammensetzungsinformations-Erfassungseinheit so konfiguriert ist, dass sie als die Informationen, die sich auf die Brustzusammensetzung beziehen, ein Verhältnis einer Brustdrüsenmasse zu dem Brustdrüsenbereich der Untersuchungsperson erfasst.

8. Ultraschallbild-Analysevorrichtung nach Anspruch 7,
wobei die Analysebestimmungseinheit (37) so konfiguriert ist, dass sie in einem Fall, in dem das Verhältnis der Brustdrüsenmasse zu dem Brustdrüsenbereich höher als ein vorbestimmter Brustdrüsenmasse-Schwellenwert ist, bestimmt, Analyse an dem Drüsengewebskomponentenbereich durchzuführen.

9. Ultraschallbild-Analysevorrichtung nach einem der Ansprüche 1-8,
wobei die die Brustdrüsenbereich-Detektionseinheit (55, 58) so konfiguriert ist, dass sie den Brustdrüsenbereich durch Bilderkennung des Ultraschallbildes detektiert.

10. Ultraschallbild-Analysevorrichtung nach Anspruch 9, ferner umfassend:
eine Brustbereichs-Detektionseinheit (54, 57), die so konfiguriert ist, dass sie einen Brustbereich, der sich zwischen einer Haut und einem großen Brustmuskel befindet, aus dem Ultraschallbild detektiert,
wobei die Brustdrüsenbereichs-Detektionseinheit (55, 58) so konfiguriert ist, dass sie eine vordere Grenzlinie und eine hintere Grenzlinie in dem Brustbereich, der von der Brustbereichs-Detektionseinheit (54, 57) detektiert wurde, erkennt und einen Bereich zwischen der vorderen Grenzlinie und der hinteren Grenzlinie als den Brustdrüsenbereich detektiert.

11. Ultraschallbild-Analysevorrichtung nach einem der Ansprüche 1-8,
wobei die Brustdrüsenbereich-Detektionseinheit (55, 58) so konfiguriert ist, dass sie durch Verwenden von Deep-Learning den Brustdrüsenbereich aus dem Ultraschallbild detektiert.

12. Ultraschallbild-Analysevorrichtung nach einem der Ansprüche 1 bis 11,
wobei die Drüsengewebskomponentenbereichs-Extraktionseinheit (59) so konfiguriert ist, dass sie den Drüsengewebskomponentenbereich extrahiert, indem sie den Brustdrüsenbereich des Ultraschallbildes durch Verwenden eines Helligkeitsschwellenwerts binarisiert.

13. Ultraschallbild-Analysevorrichtung nach einem der Ansprüche 1 bis 11,
wobei die Drüsengewebskomponentenbereichs-Extraktionseinheit (59) so konfiguriert ist, dass sie durch Verwenden von Deep-Learning den Drüsengewebskomponentenbereich aus dem Brustdrüsenbereich des Ultraschallbildes extrahiert.

14. Ultraschallbild-Analysevorrichtung nach einem der Ansprüche 1 bis 13,
wobei die Drüsengewebskomponentenbereichs-Verhältnis-Berechnungseinheit (60) so konfiguriert ist, dass sie das Verhältnis des Drüsengewebskomponentenbereichs zu dem Brustdrüsenbereich auf der Grundlage der Anzahl an belegten Pixeln des Brustdrüsenbereichs und der Anzahl an belegten Pixeln des Drüsengewebskomponentenbereichs in dem Ultraschallbild berechnet.

15. Ultraschallbild-Analysevorrichtung nach einem der Ansprüche 1 bis 14,
wobei das Ultraschallbild ein dreidimensionales Ultraschallbild ist, und
die Drüsengewebskomponentenbereichs-Verhältnis-Berechnungseinheit (60) so konfiguriert ist, dass sie das Verhältnis des Drüsengewebskomponentenbereichs zu dem Brustdrüsenbereich auf der Grundlage eines Volumens des Brustdrüsenbereichs, das von der Brustdrüsenbereichs-Detektionseinheit (55, 58) detektiert wurde, und eines Volumens des Drüsengewebskomponentenbereichs, der von der Drüsengewebskomponentenbereichs-Extraktionseinheit (59) extrahiert wurde, berechnet.

16. Ultraschalldiagnosevorrichtung, umfassend:
einen Monitor (33), der so konfiguriert ist, dass er ein Ultraschallbild anzeigt; und
die Ultraschallbild-Analysevorrichtung (4) nach einem der Ansprüche 1 bis 15,
wobei die Drüsengewebskomponentenbereichs-Analyseeinheit (38) so konfiguriert ist, dass sie ein Analyseergebnis des Drüsengewebekomponentenbereichs auf dem Monitor (33) anzeigt.

17. Ultraschalldiagnosevorrichtung nach Anspruch 16,
wobei die Drüsengewebskomponentenbereichs-Analyseeinheit (38) so konfiguriert ist, dass sie das Analyseergebnis des Drüsengewebskomponentenbereichs in einem Etikett, das dem Ultraschallbild zugeordnet ist, speichert.

18. Ultraschalldiagnosevorrichtung nach Anspruch 16 oder 17, ferner umfassend:
eine Ultraschallsonde (2); und
eine Bilderzeugungseinheit (31), die so konfiguriert ist, dass sie das Ultraschallbild, das durch Abbilden der Brust der Untersuchungsperson durch Transmittieren und Empfangen eines Ultraschallstrahls zu und von der Untersuchungsperson unter Verwendung der Ultraschallsonde (2) erhalten wird, erzeugt.

19. Computerimplementiertes Steuerverfahren für eine Ultraschallbild-Analysevorrichtung, wobei das Verfahren umfasst:
Erfassen von Informationen, die sich auf eine Brustzusammensetzung einer Untersuchungsperson, die sich auf eine Brustzusammensetzung einer Untersuchungsperson beziehen und durch eine Mammographieuntersuchung erhalten wurden;
Bestimmen auf der Grundlage der erfassten Informationen, die sich auf die Brustzusammensetzung beziehen, ob Analyse an einem Drüsengewebskomponentenbereich, der Milchgänge, Läppchen und peripheres Stroma enthält, in einem Brustdrüsenbereich durchgeführt werden soll oder nicht;
**gekennzeichnet durch**
Durchführen von Analyse an dem Drüsengewebekomponentenbereich auf der Grundlage eines Ultraschallbildes, das durch Abbilden einer Brust der Untersuchungsperson in einem Fall, in dem bestimmt wird, Analyse durchzuführen, erhalten wird;
wobei die Analyse an dem Drüsengewebskomponentenbereich durchgeführt wird:
durch
Detektieren des Brustdrüsenbereichs aus dem Ultraschallbild;
Extrahieren des Drüsengewebskomponentenbereichs in dem detektierten Brustdrüsenbereich; und
Berechnen des Verhältnisses des Drüsengewebskomponentenbereichs zu dem Brustdrüsenbereich.

## Revendications

1. Appareil d'analyse d'images ultrasonores comprenant :
une unité d'acquisition d'informations de composition de sein qui est configurée pour acquérir des informations liées à une composition de sein d'un sujet obtenue par un examen de mammographie ;
une unité de détermination d'analyse (37) qui est configurée pour déterminer si oui ou non une analyse sur une région de composant de tissu glandulaire incluant des canaux mammaires, des lobules et des stromas périphériques dans une région de glande mammaire sur la base des informations liées à la composition de sein acquises par l'unité d'acquisition d'informations de composition de sein (36) doit être effectuée ; et
une unité d'analyse de région de composant de tissu glandulaire (38) qui est configurée pour effectuer une analyse sur la région de composant de tissu glandulaire sur la base d'une image ultrasonore obtenue en imageant un sein du sujet dans un cas où l'unité de détermination d'analyse (37) détermine d'effectuer une analyse ;
**caractérisé en ce que**
l'unité d'analyse de région de composant de tissu glandulaire (38) est configurée pour calculer un rapport de la région de composant de tissu glandulaire à la région de glande mammaire ; et
**en ce que**
l'unité d'analyse de région de composant de tissu glandulaire (38) inclut :
une unité de détection de région de glande mammaire (55, 58) qui est configurée pour détecter la région de glande mammaire à partir de l'image ultrasonore ;
une unité d'extraction de région de composant de tissu glandulaire (59) qui est configurée pour extraire la région de composant de tissu glandulaire dans la région de glande mammaire détectée par l'unité de détection de région de glande mammaire (55, 58) ; et
une unité de calcul de rapport de région de composant de tissu glandulaire (60) qui est configurée pour calculer le rapport de la région de composant de tissu glandulaire à la région de glande mammaire.

2. Appareil d'analyse d'images ultrasonores selon la revendication 1,
dans lequel l'unité d'acquisition d'informations de composition de sein est configurée pour acquérir, en tant que les informations liées à la composition de sein, une catégorie de glande mammaire correspondant au sujet parmi des catégories de glande mammaire classées en une pluralité de types en fonction d'une densité de glande mammaire.

3. Appareil d'analyse d'images ultrasonores selon la revendication 2,
dans lequel l'unité de détermination d'analyse (37) est configurée pour déterminer d'effectuer une analyse sur la région de composant de tissu glandulaire dans un cas où la catégorie de glande mammaire correspondant au sujet appartient à au moins une catégorie de glande mammaire qui est déterminée dans un ordre dans lequel la densité de glande mammaire est élevée parmi les catégories de glande mammaire classées en la pluralité de types.

4. Appareil d'analyse d'images ultrasonores selon la revendication 1,
dans lequel l'unité d'acquisition d'informations de composition de sein est configurée pour acquérir, en tant que les informations liées à la composition de sein, un rapport de la région de glande mammaire à une région de sein du sujet.

5. Appareil d'analyse d'images ultrasonores selon la revendication 4,
dans lequel l'unité d'acquisition d'informations de composition de sein inclut une unité de calcul d'informations de composition de sein (36) qui est configurée pour calculer le rapport de la région de glande mammaire à la région de sein du sujet à partir d'une image de mammographie.

6. Appareil d'analyse d'images ultrasonores selon la revendication 4 ou la revendication 5,
dans lequel l'unité de détermination d'analyse (37) est configurée pour déterminer d'effectuer une analyse sur la région de composant de tissu glandulaire dans un cas où le rapport de la région de glande mammaire à la région de sein est supérieur à une valeur seuil de région de glande mammaire prédéterminée.

7. Appareil d'analyse d'images ultrasonores selon la revendication 1,
dans lequel l'unité d'acquisition d'informations de composition de sein est configurée pour acquérir, en tant que les informations liées à la composition de sein, un rapport d'une masse de glande mammaire à la région de glande mammaire du sujet.

8. Appareil d'analyse d'images ultrasonores selon la revendication 7,
dans lequel l'unité de détermination d'analyse (37) est configurée pour déterminer d'effectuer une analyse sur la région de composant de tissu glandulaire dans un cas où le rapport de la masse de glande mammaire à la région de glande mammaire est supérieur à une valeur seuil de masse de glande mammaire prédéterminée.

9. Appareil d'analyse d'images ultrasonores selon l'une quelconque des revendications 1-8,
dans lequel l'unité de détection de région de glande mammaire (55, 58) est configurée pour détecter la région de glande mammaire par reconnaissance d'image de l'image ultrasonore.

10. Appareil d'analyse d'images ultrasonores selon la revendication 9, comprenant en outre :
une unité de détection de région de sein (54, 57) qui est configurée pour détecter une région de sein située entre une peau et un muscle pectoral majeur à partir de l'image ultrasonore,
dans lequel l'unité de détection de région de glande mammaire (55, 58) est configurée pour reconnaître une ligne limite avant et une ligne limite arrière dans la région de sein détectée par l'unité de détection de région de sein (54, 57), et détecter une région entre la ligne limite avant et la ligne limite arrière, en tant que la région de glande mammaire.

11. Appareil d'analyse d'images ultrasonores selon l'une quelconque des revendications 1-8,
dans lequel l'unité de détection de région de glande mammaire (55, 58) est configurée pour détecter la région de glande mammaire à partir de l'image ultrasonore en utilisant l'apprentissage profond.

12. Appareil d'analyse d'images ultrasonores selon l'une quelconque des revendications 1 à 11,
dans lequel l'unité d'extraction de région de composant de tissu glandulaire (59) est configurée pour extraire la région de composant de tissu glandulaire en binarisant la région de glande mammaire de l'image ultrasonore en utilisant une valeur seuil de luminosité.

13. Appareil d'analyse d'images ultrasonores selon l'une quelconque des revendications 1 à 11,
dans lequel l'unité d'extraction de région de composant de tissu glandulaire (59) est configurée pour extraire la région de composant de tissu glandulaire à partir de la région de glande mammaire de l'image ultrasonore en utilisant l'apprentissage profond.

14. Appareil d'analyse d'images ultrasonores selon l'une quelconque des revendications 1 à 13,
dans lequel l'unité de calcul de rapport de région de composant de tissu glandulaire (60) est configurée pour calculer le rapport de la région de composant de tissu glandulaire à la région de glande mammaire sur la base du nombre de pixels occupés de la région de glande mammaire et du nombre de pixels occupés de la région de composant de tissu glandulaire dans l'image ultrasonore.

15. Appareil d'analyse d'images ultrasonores selon l'une quelconque des revendications 1 à 14,
dans lequel l'image ultrasonore est une image ultrasonore tridimensionnelle, et
l'unité de calcul de rapport de région de composant de tissu glandulaire (60) est configurée pour calculer le rapport de la région de composant de tissu glandulaire à la région de glande mammaire sur la base d'un volume de la région de glande mammaire détecté par l'unité de détection de région de glande mammaire (55, 58) et d'un volume de la région de composant de tissu glandulaire extrait par l'unité d'extraction de région de composant de tissu glandulaire (59).

16. Appareil de diagnostic par ultrasons comprenant :
un moniteur (33) qui est configuré pour afficher une image ultrasonore ; et
l'appareil d'analyse d'images ultrasonores (4) selon l'une quelconque des revendications 1 à 15,
dans lequel l'unité d'analyse de région de composant de tissu glandulaire (38) est configurée pour afficher un résultat d'analyse de la région de composant de tissu glandulaire sur le moniteur (33).

17. Appareil de diagnostic par ultrasons selon la revendication 16,
dans lequel l'unité d'analyse de région de composant de tissu glandulaire (38) est configurée pour stocker le résultat d'analyse de région de composant de tissu glandulaire dans une étiquette associée à l'image ultrasonore.

18. Appareil de diagnostic par ultrasons selon la revendication 16 ou la revendication 17, comprenant en outre :
une sonde ultrasonore (2) ; et
une unité de génération d'images (31) qui est configurée pour générer l'image ultrasonore obtenue en imageant le sein du sujet en transmettant et en recevant un faisceau ultrasonore vers le sujet et à partir de celui-ci en utilisant la sonde ultrasonore (2).

19. Procédé de contrôle implémenté par ordinateur pour un appareil d'analyse d'images ultrasonores, le procédé comprenant
acquérir des informations liées à une composition de sein d'un sujet obtenue par un examen de mammographie ;
déterminer si oui ou non une analyse sur une région de composant de tissu glandulaire incluant des canaux mammaires, des lobules et des stromas périphériques dans une région de glande mammaire sur la base des informations acquises liées à la composition de sein doit être effectuée ;
**caractérisé par**
effectuer une analyse sur la région de composant de tissu glandulaire sur la base d'une image ultrasonore obtenue en imageant un sein du sujet dans un cas où il est déterminé d'effectuer une analyse ;
dans lequel l'analyse de la région de composant de tissu glandulaire est effectuée par :
détecter la région de glande mammaire à partir de l'image ultrasonore ;
extraire la région de composant de tissu glandulaire dans la région de glande mammaire détectée ; et
calculer le rapport de la région de composant de tissu glandulaire à la région de glande mammaire.
